# FASCICULE DE BREVET EUROPEEN

(11) **EP 4 408 363 B1**
(45) Date de publication et mention de la délivrance du brevet: **27.11.2024**
(21) Numéro de dépôt: 22792850.4
(22) Date de dépôt: 23.09.2022
(51) Int. Cl.: A61F 13/00, A61F 13/42, G01M 3/16, G01N 27/12

(54) **CAPTEUR D'HUMIDITÉ**
FEUCHTIGKEITSSENSOR
MOISTURE SENSOR

(30) Priorité: 30.09.2021 FR 2110303
(43) Date de publication de la demande: 07.08.2024
(73) Titulaire: Linxens Holding, 78200 Mantes-la-Jolie (FR)
(72) Inventeur: VASSAL, Simon, 78200 Mantes-la-Jolie (FR); MOUSQUE, Valerie, 78200 Mantes-la-Jolie (FR); GERMAIN, Francois, 78200 Mantes-la-Jolie (FR); PRUNIER, Sebastien, 78200 Mantes-la-Jolie (FR)
(74) Mandataire: Grünecker Patent- und Rechtsanwälte PartG mbB
(86) Numéro de dépôt international: PCT/FR2022/051790
(87) Numéro de publication internationale: WO 2023/052711

(56) Documents cités:
- EP-A1- 2 014 267
- EP-A1- 2 944 299
- WO-A1-2013/114273
- US-A1- 2020 405 546

## Description

La présente invention se rapporte à un capteur d'humidité destiné à détecter la présence et la position d'un liquide sur une surface.

Le liquide est issu d'un point d'origine, le capteur d'humidité permet de mesurer l'étendue de l'écoulement dans un plan.

Grâce à un système d'électrodes et de composants électronique choisis spécifiquement, il est possible de détecter la localisation d'un liquide sur une surface donnée. Les applications sont multiples : détection de fuite, vêtement intelligent, pansement, couches, capteur de niveau, mobilité (voiture, bateau, etc.).

En particulier, la présente invention est particulièrement adaptée pour être intégrée dans un pansement pour une plaie chronique, dont la cicatrisation peut prendre jusqu'à 12 mois, voire 18 mois.

Les plaies suintent un liquide, appelé exsudat, qui contient - entre autres - de l'eau et des électrolytes. Ce liquide, ou exsudat, mouille progressivement le pansement. Lorsque le pansement atteint un certain niveau de remplissage, il faut changer le pansement. Le pansement peut être changé à intervalle régulier. Toutefois, lorsque le niveau de remplissage n'est pas atteint, le changement de pansement occasionne des frais (liés au coût du personnel soignant et du pansement) et une mise à l'air de la plaie, qu'il serait avantageux d'éviter.

C'est pourquoi il est connu de l'état de la technique d'équiper les pansements de systèmes de détection d'humidité afin de déduire l'état de remplissage du pansement. Il est notamment connu de WO 2013/114273, d'évaluer le remplissage d'un pansement de plaie en mesurant la variation de la conductivité électrique entre deux électrodes successives d'un système de détection d'humidité du pansement. Un tel système de détection d'humidité du pansement est donc dépendant de la conductivité électrique du liquide. Ce système s'adapte mal aux différences de conductivité électriques des différents liquides pouvant être exsudés (sang, pus, etc..) qui dépendent de la pathologie, de la plaie et des données physiologiques du patient.

De l'état de la technique antérieure, on connait également des capteurs d'humidité décrits dans les documents EP 2 014 267, EP 2 944 299, et US 2020/405546.

L'objet de la présente invention est de proposer un capteur d'humidité amélioré par rapport à ceux connus de l'état de la technique.

L'objet de la présente invention est atteint au moyen d'un capteur d'humidité selon l'énoncé de la revendication indépendante 1 comprenant un substrat de support ayant une première face et une deuxième face, géométriquement opposée à la première face. La première face est pourvue d'une pluralité de couples d'électrodes configurés pour être en contact avec un liquide. La deuxième face comprend une pluralité de premiers moyens de détection connectés entre eux en parallèle, et chaque premier moyen de détection comprend un transistor associé à un couple d'électrodes. Ledit transistor de chaque premier moyen de détection est configuré pour commuter d'un premier état vers un second état lors du passage d'un courant électrique entre des électrodes du couple d'électrodes associé au transistor. Une électrode du couple d'électrodes est électriquement connectée en série à une électrode d'entrée du transistor et l'autre électrode du couple d'électrodes est électriquement connectée à un potentiel fixe, en particulier à un potentiel à la masse, et une électrode de sortie du transistor étant électriquement connecté en série avec une première résistance, de sorte que le capteur d'humidité est sensible à la résistance équivalente desdites premières résistances de la pluralité des premiers moyens de détection.

Ainsi, la détection d'humidité n'est pas directement réalisée à partir de la mesure de la résistance, l'impédance ou de la conductivité électrique du liquide en contact avec les électrodes.

Dans la présente invention, il n'est pas nécessaire de mesurer la variation de la résistance aux bornes des électrodes de chaque couple d'électrodes, le courant électrique circulant à travers le couple d'électrodes étant utilisé pour faire commuter, ou non, les transistors. Le courant électrique est induit par la présence de liquide conducteur aux bornes des électrodes, indépendamment des propriétés spécifiques du liquide. La détection d'humidité est alors basée sur un nombre de transistors commutés, lesdits transistors étant commutables entre deux états.

De la résistance équivalente peut être déduit le nombre de premières résistances associées à chaque transistor qui a été commuté par la présence de liquide entre les électrodes d'un couple d'électrodes. Il est ainsi possible d'en déduire le nombre de couples d'électrodes connectées par la présence de liquide entre les électrodes du couple d'électrodes. Le nombre de couples d'électrodes connectées est relatif à la surface mouillée. La résistance équivalente selon la présente invention est donc corrélée à la présence de liquide entre les électrodes de la première face.

De ce fait, la présente invention permet avantageusement de s'affranchir de la mesure de la résistance (ou de l'impédance ou de la conductivité électrique) du liquide qui dépend de la composition et des propriétés propres à chaque liquide, en particulier à chaque exsudat dans le cas d'une application du capteur d'humidité dans un pansement.

En particulier, le capteur d'humidité peut être configuré pour mesurer la résistance équivalente des premières résistances de la pluralité des premiers moyens de détection.

Plus précisément, le capteur d'humidité peut comprendre un circuit intégré ou une puce électronique, notamment pour mesurer la résistance équivalente et, éventuellement, pour traiter les données mesurées.

Par ailleurs, le capteur d'humidité peut-être un appareil électrique qui peut comprendre au moins une unité de calcul et/ou un processeur. En plus, ou à la place de l'unité de calcul et/ou du processeur, le capteur d'humidité peut comprendre un ordinateur intégré (« *embedded computer* » en anglais).

Au moyen de l'unité de calcul et/ou du processeur et/ou de l'ordinateur intégré, le capteur d'humidité peut être configuré pour mesurer la résistance équivalente des premières résistances de la pluralité des premiers moyens de détection.

Le capteur d'humidité pourrait aussi être configuré pour déterminer la tension aux bornes des premières résistances. Dans ce cas, lorsqu'une tension est mesurée, le capteur d'humidité peut en outre comprendre des moyens amplificateurs afin d'amplifier la tension mesurée avant d'être traitée par une unité de calcul, un processeur et/ou un ordinateur intégré.

Le capteur d'humidité peut également comprendre des moyens de stockage de donnée. Le support de stockage peut être sélectionné par l'homme du métier parmi les supports de stockage connus, par exemple selon leur capacité et leur vitesse. Par exemple, des valeurs de résistance équivalente mesurée et/ou de tension obtenue peuvent être stockées dans ces moyens de stockage de données.

En outre, il convient de noter que la connexion d'une des électrodes à un potentiel fixe permet de faciliter la détermination du nombre de transistor commutés dans un desdits états par rapport à ceux commutés dans l'autre état.

En particulier, la connexion d'une électrode à un potentiel fixe et la connexion de l'autre électrode en série à l'électrode d'entrée du transistor permettent d'obtenir une variation de la tension par palier, facilitant la détection d'humidité.

Le capteur d'humidité selon la présente invention peut être davantage amélioré grâce aux modes de réalisation suivants.

Selon un mode de réalisation de l'invention, le transistor peut être un transistor bipolaire, en particulier un transistor bipolaire de type positif-négatif-positif (PNP), configuré pour commuter d'un état dit passant, correspondant au premier état, vers un état dit bloqué, correspondant au second état, et dans lequel l'électrode d'entrée du transistor est une base du transistor bipolaire et l'électrode de sortie du transistor est un collecteur du transistor bipolaire.

Selon un mode de réalisation de l'invention, le transistor peut être un transistor à effet de champ à grille isolée (MOSFET) dans lequel l'électrode d'entrée du transistor est une grille du transistor MOSFET et l'électrode de sortie du transistor est un drain du transistor MOSFET.

Selon un mode de réalisation de l'invention, le capteur d'humidité peut comprendre des premières résistances de valeurs différentes.

Il est possible d'améliorer la sensibilité et la qualité de la détection d'humidité en utilisant des premières résistances de valeurs différentes.

Selon un mode de réalisation de l'invention, la pluralité de couples d'électrodes peut être répartie par zones prédéfinies de la première face, et la valeur de chaque première résistance peut être relative à une zone prédéfinie de la première face.

Il est ainsi possible de déterminer non seulement la présence, mais aussi la répartition de liquide sur les différentes zones prédéfinies du capteur d'humidité.

Par exemple, une valeur de première résistance associée aux couples d'électrodes disposés de manière centrale sur la première face peut être différente d'une valeur de première résistance associée à un couple d'électrodes disposé de manière périphérique sur la première face. Cela permet de déterminer si le centre de la première face est davantage mouillé ou pas que sa périphérie.

Selon un mode de réalisation de l'invention, la pluralité de premiers moyens de détection peut être enrobée dans une résine d'encapsulation.

La résine d'encapsulation permet d'assurer l'étanchéité et d'éviter ainsi des courts circuits.

Selon un mode de réalisation de l'invention, le substrat de support peut comprendre au moins un orifice traversant permettant un écoulement d'un fluide entre la première face et la deuxième face.

La présence d'au moins un orifice traversant permet au liquide de traverser le substrat de support, qui ainsi n'est pas rendu imperméable. L'orifice traversant permet la diffusion « verticale » du liquide à travers le substrat de support selon une direction s'étendant de la première face vers la deuxième face.

Selon un mode de réalisation de l'invention, les électrodes de la pluralité de couples d'électrodes peuvent être disposées sur la première face selon au moins deux directions contenues dans un plan de la première face, en particulier en croix, en particulier en croix à au moins six branches.

La disposition des électrodes en croix, ou en étoile, permet de faciliter la diffusion « horizontale » du liquide dans le plan de la première face. En effet, les électrodes, selon leur agencement, pourraient bloquer la propagation du liquide dans le plan de la première face.

Selon un mode de réalisation de l'invention, pour chaque couple d'électrodes, chacune des électrodes du couple d'électrodes peut être électriquement connectée par un trou métallisé formé entre la première face et la deuxième face à un premier moyen de détection correspondant qui est disposé sur ladite deuxième face.

Selon un mode de réalisation de l'invention, le substrat de support peut être une couche flexible apte à être courbée, en particulier de moins de 300 micromètres d'épaisseur, plus particulièrement de moins de 150 micromètres d'épaisseur.

Selon un mode de réalisation de l'invention, le capteur d'humidité peut en outre comprendre une puce électronique au niveau de la deuxième face configurée pour transmettre des données à un dispositif tiers, notamment par une communication radiofréquence.

Ainsi, le capteur d'humidité est adapté pour pouvoir traiter les données recueillies par les moyens de détections et de les communiquer par radiofréquence.

Selon un mode de réalisation de l'invention, la première face peut comprendre en outre une antenne radioélectrique.

Ainsi, les données issues du système de détection d'humidité peuvent être communiquées à un dispositif tiers par radiofréquence.

Le fait que l'antenne radioélectrique soit disposée sur la première face, c'est-à-dire sur la face pourvue avec les électrodes, permet non seulement un gain de place mais aussi de faciliter le montage et les connexions électriques dans le substrat de support.

Selon un mode de réalisation de l'invention, l'antenne radioélectrique peut être disposée autour de pluralité des couples d'électrodes.

Ainsi, il est possible de facilement fabriquer des capteurs d'humidités de différentes formes, sans se soucier de la distance inter-électrodes par exemple.

Selon un mode de réalisation de l'invention, le substrat de support peut en outre comprendre au moins un autre couple d'électrodes de détection et au moins un deuxième moyen de détection d'une autre grandeur que l'humidité, et ledit couple d'électrodes de détection étant électriquement connecté au deuxième moyen de détection de sorte que le capteur d'humidité est en outre configuré pour mesurer une autre grandeur que l'humidité, en particulier la température, la pression, le pH ou la présence d'une espèce chimique.

Le capteur d'humidité peut ainsi avantageusement permettre la détection de grandeurs physiques, chimiques, voire électrochimiques, en addition à la détection d'humidité. L'utilisation de différents capteurs distincts peut ainsi être évitée en regroupant plusieurs moyens de détections dans le capteur d'humidité.

Le ou les deuxièmes moyen(s) de détection peut/peuvent être enrobé(e) dans la résine d'encapsulation.

L'objet de la présente invention se rapporte aussi, selon l'énoncé de la revendication indépendante 15, à l'utilisation du capteur d'humidité décrit ci-dessus, dans un pansement de plaie dans lequel la pluralité de couples d'électrodes du capteur d'humidité est agencé dans le pansement pour être en contact avec un liquide provenant d'une plaie.

L'utilisation du capteur d'humidité dans un pansement permet avantageusement de s'affranchir de la mesure de la résistance (ou de l'impédance ou de la conductivité électrique) du sang, exsudat, pu, etc. qui dépend particulièrement de chaque plaie et patient.

L'objet de la présente invention est aussi atteint, selon l'énoncé de la revendication indépendante 16, au moyen d'un procédé de détection d'humidité au moyen d'un capteur d'humidité selon l'un des modes de réalisation mentionnés ci-dessus, comprenant les étapes de a) mesurer la résistance équivalente des premières résistances de la pluralité des premiers moyens de détection du capteur d'humidité qui est dépendante du nombre de transistors commutés dans un des premiers ou second états, puis b) déduire de la valeur de la résistance équivalente mesurée la présence de liquide sur la première face pourvue des électrodes.

Le procédé de détection d'humidité selon la présente invention permet avantageusement de s'affranchir de la mesure de la résistance (ou de l'impédance ou de la conductivité électrique) du liquide qui dépend de la composition et des propriétés propres à chaque liquide, en particulier à chaque exsudat dans le cas d'une application du capteur d'humidité dans un pansement.

Les modes de réalisation peuvent être combinés pour former davantage de variantes de mode de réalisation avantageux de la présente invention.

L'invention et ses avantages seront expliqués plus en détail dans la suite au moyen de modes de réalisation préférés et en s'appuyant notamment sur les figures d'accompagnement suivantes, dans lesquelles :
Fig. 1 est une vue schématique en coupe d'un capteur d'humidité selon un mode de réalisation de la présente invention,
Fig. 2 est une vue schématique en coupe d'un pansement dans lequel est intégré le capteur d'humidité selon un mode de réalisation illustré à la Figure 1,
Fig. 3 est une vue schématique d'une première face d'un capteur d'humidité selon un mode de réalisation de la présente invention,
Fig. 4 est une vue schématique du capteur d'humidité de la Figure 3 comprenant une antenne radiofréquence selon une variante de l'invention,
Fig. 5 représente schématiquement un moyen de détection selon un mode de réalisation de la présente invention,
Fig. 6 représente schématiquement l'association de premières résistances en parallèle du système de détection d'humidité selon la présente invention,
Fig. 7 représente l'évolution de la tension en fonction du nombre de couples d'électrodes connectées.
Fig. 8 représente un système comprenant un pansement tel qu'illustré à la Figure 2 et un dispositif de communication,
Fig. 9 représente le système illustré à la Figure 8 et un terminal utilisateur.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

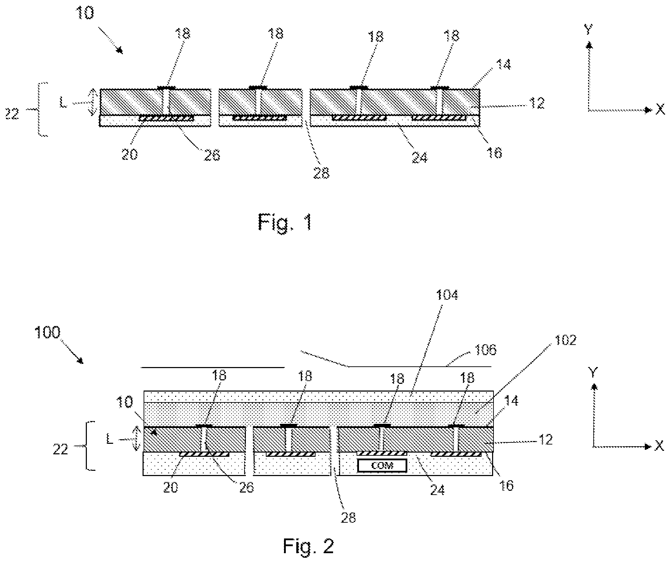

La Figure 1 est une vue schématique en coupe d'un capteur d'humidité 10 selon un mode de réalisation de la présente invention.

Le capteur d'humidité 10 est destiné à détecter la présence et la position d'un liquide sur une surface.

Le capteur d'humidité 10 comprend un substrat de support 12.

Le substrat de support 12 est une couche flexible apte à être courbée, en particulier afin de pouvoir se conformer à la courbure d'un élément.

Par exemple, dans le cas où le capteur d'humidité 10 est intégré dans un pansement, le substrat de support flexible 12 est adapté pour être courbé de manière à épouser la forme d'une partie d'un corps humain sur laquelle est prévue d'être appliquée ledit pansement.

Le substrat de support 12 peut être réalisé en verre époxy, en polyimide, en polyéthylène téréphtalate (PET), polyéthylène naphtalène (PEN) ou autre.

Le substrat de support 12 a une épaisseur L de moins de 300 micromètres d'épaisseur, en particulier de moins de 150 micromètres d'épaisseur. Il est noté que l'épaisseur L correspond à l'épaisseur du substrat de support 12 seul, sans prendre en considération l'épaisseur d'une quelconque autre couche que le substrat 12 lui -même, comme un encapsulant.

Le substrat de support 12 comprend une première face 14 et une deuxième face 16, géométrique opposée à la première face 14 selon un axe Y du repère de coordonnées Cartésien.

La première face 14 est pourvue d'une pluralité électrodes 18 configurés pour être en contact avec un liquide.

Les électrodes 18 sont préférentiellement en alliage de cuivre plaqué or ou nickel-or, en aluminium, en bronze, en or, plaqué or ou autre.

La deuxième face 16 est pourvue d'une pluralité de moyens de détection 20. Tel que décrit ci-après les moyens de détection 20 sont connectés entre eux en parallèle. Les moyens de détection 20 peuvent aussi être désignés dans ce qui suit comme « premiers moyens de détection 20 ». Les premiers moyens de détection 20 sont configurés pour détecter l'humidité. Tel qu'il sera expliqué ci-après, dans une variante, le substrat de support 12 peut en outre comprendre un ou des deuxième(s) moyen(s) de détection (non représenté) configuré(s) pour détecter une autre grandeur que l'humidité, notamment pour mesurer la température, la pression, le pH ou la présence d'une certaine espèce chimique.

L'ensemble du substrat de support 12, des électrodes 18 et des moyens de détection 20 sont constituées par un circuit imprimé électrique 22.

Le circuit imprimé électrique 22 peut être fabriqué à partir de procédés connus de fabrication de circuits imprimés souples, c'est-à-dire flexibles, aussi connus sous la dénomination de « flex PCB » ou « circuit flex », notamment dans le domaine des cartes à puce. En particulier, le circuit imprimé électrique 22 peut être fabriqué à partir du procédé de fabrication divulgué dans la demande de brevet française FR 3013504 A1.

Selon un tel procédé de fabrication, un matériau formé de la couche de substrat de support 12 et d'au moins une feuille de matériau électriquement conducteur est fourni sur sa première face 14. Le matériau conducteur est par exemple du cuivre, de l'aluminium ou un alliage de cuivre ou d'aluminium. La deuxième face 16 du substrat de support 12 est enduite avec un matériau adhésif (non représenté). Le substrat de support 12 revêtu de l'adhésif et du matériau conducteur est perforé, par exemple par poinçonnage mécanique ou par laser, pour former des trous 26 et éventuellement des ouvertures (perforations) latérales utilisées pour le guidage d'une bande dans un procédé de bobine à bobine. Une deuxième feuille de matériau électriquement conducteur est ensuite laminée sur la deuxième face 16 du substrat de support 12. Cette feuille recouvre ainsi l'adhésif et les trous 26. Les trous 26 sont alors des trous dit « borgnes » car fermés d'un côté par le feuillet métallique.

Alternativement, on fournit un matériau formé du substrat de support 12 et de deux couches de matériaux conducteur. Ce matériau est perforé, par exemple par poinçonnage mécanique ou par laser, pour former des trous 26, qui sont alors des trous dit « traversants ».

Que les trous 26 soient formés de manière à être traversants ou non, une ou plusieurs couches de matériau électriquement conducteur sont alors électro-déposées dans les trous 26. Des motifs sont ensuite réalisés par photolithographie sur les deux feuilles de matériau électriquement conducteur par dépôt, insolation et révélation d'une résine photosensible.

Une étape de gravure de motifs par voie chimique permet de réaliser notamment les électrodes 18 sur la face 14 et le circuit électrique 22 permettant de connecter les moyens de détection 20 sur la face 16.

La résine protégeant les motifs pendant la gravure est retirée chimiquement et des couches de finition (Nickel et Or par exemple) sont éventuellement déposées par voie électrochimique ou chimique sur les motifs gravés et dans les trous métallisés 26.

Les composants électroniques utilisés dans les moyens de détection 20 sont ensuite assemblés sur le circuit électrique 22.

Afin d'isoler électriquement le circuit électrique 22 et les moyens de détection 20 d'un liquide, le circuit électrique 22 et les moyens de détection 20 sont enrobés dans une résine d'encapsulation 24 au niveau de la deuxième face 16. La résine 24 d'encapsulation permet d'assurer l'étanchéité au niveau de la deuxième face 16 et d'éviter des courts circuits.

La résine d'encapsulation 24 peut être une résine silicone, polyamide, époxy ou autre.

Tel qu'illustré à la Figure 1, chacune des électrodes 18 est reliée électriquement à un moyen de détection 20 correspondant par un trou métallisé 26 traversant le substrat de support 12 de la première face 12 à la deuxième face 16.

Autrement dit, chacun des trous métallisés 26 permet de connecter électriquement une électrode 18 à un moyen de détection 20.

Tel qu'expliqué ci-dessus au regard du procédé de fabrication, les trous 26 sont métallisés par un dépôt de cuivre, de nickel et/ou d'or.

Tel qu'illustré à la Figure 1, le circuit électrique 22 est pourvu d'au moins un orifice traversant 28 permettant un écoulement d'un fluide entre la première face 14 et la deuxième face 16. Chaque orifice traversant 28 s'étend ainsi de la première face 14 à la deuxième face 16 à travers le circuit électrique 22 et la résine d'encapsulation 24.

Chaque orifice traversant 28 peut être réalisé par poinçonnage ou perçage à travers la résine d'encapsulation et le circuit électrique 22.

La Figure 2 représente schématiquement l'intégration d'un capteur d'humidité 10 selon le mode de réalisation illustré à la Figure 1 dans un pansement 100. Le pansement 100 comprend le capteur d'humidité 10 et un module de communication COM.

Les éléments avec les mêmes références numériques déjà utilisées pour la description de la Figure 1 ne seront pas décrits à nouveau en détail, et référence est faite à leurs descriptions ci-dessus.

Le pansement 100 comprend une pluralité de couches. Certaines couches ont été volontairement omises dans la représentation schématique de la figure 2 par souci de clarté et de simplification du dessin.

Une couche absorbante 102 est fournie sur première face 14 du substrat de support 12 du capteur d'humidité 10. La couche absorbante 102 peut elle-même être constituée d'une pluralité de couches (non-représentée). La couche absorbante 102 comprend une face d'application 104 configurée pour être appliquée sur une plaie. La couche absorbante 102 est ainsi configurée pour absorber un liquide, en particulier un exsudat, provenant d'une plaie sur laquelle le pansement 100 est destiné à être appliqué. L'exsudat est un liquide qui suinte d'une plaie. L'exsudat contient entre autres de l'eau et des électrolytes. Les électrodes 18 étant disposées sur la première face 14, les électrodes 18 sont configurées pour être en contact avec un liquide absorbé par la couche absorbante 102.

Tel qu'expliqué en référence à la Figure 1, la résine d'encapsulation 24 permet d'assurer l'étanchéité au niveau de la deuxième face 16 et d'éviter des courts circuits. Toutefois l'étanchéité au niveau de la deuxième face 16 a pour effet de rendre le substrat de support 12 non-perméable. Or, il est nécessaire que le liquide provenant de la plaie puisse se diffuser à travers le pansement 100, notamment de manière verticale c'est-à-dire dans une direction de profondeur du pansement 100 le long de l'axe Y représenté sur la figure 2. À cet effet, les orifices traversant 28 permettent une communication fluidique entre la première face 14 et la deuxième face 16. La répartition des orifices traversant 28 peut être régulière ou varier entre une zone centrale du pansement 100 et une partie périphérique du pansement 100.

Pour communiquer avec au moins un dispositif tiers, le pansement 100 comprend le module de communication COM. Dans l'exemple du mode de réalisation illustré par la Figure 2, le module de communication COM est encapsulé dans la résine 24 pour qu'il soit protégé. Alternativement, le module de communication COM peut être partiellement encapsulé.

Le module de communication COM peut comprendre un circuit intégré, par exemple un processeur, et une antenne. Le module de communication COM peut notamment être un module de communication en champ proche (« Near Field Communication (NFC) » en anglais).

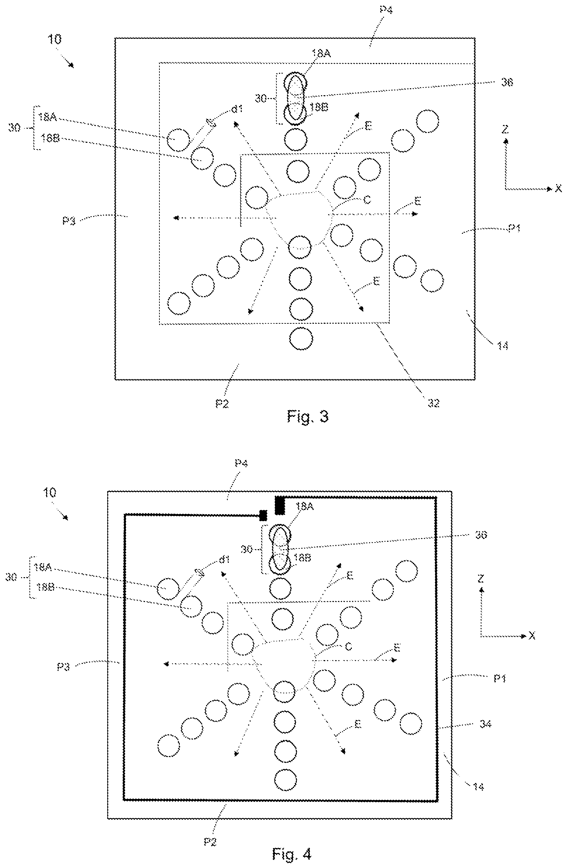

Sur la Figure 2, le module de communication COM est représenté schématiquement par un bloc, sans que le circuit intégré ou l'antenne ne soient visibles, et sans que cette représentation ne soit limitative. Le module de communication COM peut être agencé de sorte que tous ses éléments sont sur une seule face (la première face 14 ou la deuxième face 16), ou répartis sur les deux faces 14 et 16. Une configuration possible du module de communication COM peut comprendre une encapsulation de son circuit intégré (non représenté sur la Figure 2) dans la résine 24, monté sur la deuxième face 16, et une installation de son antenne (non représenté sur la Figure 2) sur la première face 14 (éventuellement sans encapsulation), comme cela sera décrit ultérieurement en référence aux Figures 3 et 4. Une connexion traversante entre l'antenne et le circuit intégré (non représentés sur la Figure 2) du module de communication COM peut être utilisée.

Enfin, la Figure 2 montre que la face d'application 104 est protégée par film de protection 106 détachable. Le pansement 100 est prévu pour être conditionné de manière stérile jusqu'à son utilisation.

La Figure 2 représente ainsi un pansement 100 de plaie comprenant le substrat de support 12 dont la première face 14 est configurée pour être appliquée, directement ou indirectement, sur une plaie. La première face 14 est pourvue de la pluralité d'électrodes 18 configurées pour être en contact avec un liquide provenant de la plaie. La deuxième face 16, qui est géométriquement opposée à la première face (14), comprend la pluralité de moyens de détection 20 connectés en parallèle. Chaque moyen de détection 20 comprend un moyen de commutation (non visible sur la Figure 2), tel qu'un transistor, associé à deux électrodes 18 formant un couple d'électrode (indiqué aux Figures 3 et 4 par la référence 30). Chaque moyen de commutation est configuré pour commuter d'un premier état vers un second état lors du passage d'un courant électrique entre le couple d'électrodes associé audit moyen de commutation. L'humidité du pansement 100, en particulier de la couche absorbante 102 peut être déterminée à partir du nombre de moyens de commutation commutés dans un desdits états par rapport à ceux commutés dans l'autre état.

La détermination de l'humidité selon la présente invention est davantage détaillée en référence aux Figures 3 à 7.

Il convient de noter que le pansement 100 peut en outre comprendre au moins un autre capteur de mesure d'une grandeur différente de l'humidité. Dans ce cas, le substrat de support 12 du capteur d'humidité 10 comprend au moins un autre couple d'électrodes de détection, en particulier distinct de la pluralité des couples d'électrodes 30 décrits ci-dessus, et au moins un deuxième moyen de détection (non représenté) d'une autre grandeur que l'humidité, ledit couple d'électrodes de détection étant électriquement connecté au deuxième moyen de détection. Ainsi, le capteur d'humidité 10 dans le pansement 100 est en outre configuré pour mesurer une autre grandeur que l'humidité, en particulier la température, la pression, le pH ou la présence d'une espèce chimique.

La Figure 3 illustre une vue schématique de la première face 14 du capteur d'humidité 10 selon un mode de réalisation de la présente invention.

Les éléments avec les mêmes références numériques déjà utilisées pour la description de la Figure 1 ne seront pas décrits à nouveau en détail, et référence est faite à leurs descriptions ci-dessus.

La première face 14 du capteur d'humidité 10 comprend la pluralité d'électrodes 18. Il est noté que la première face 14 ne comprend pas de composants électroniques, ceux-ci sont disposés sur la deuxième face 16 et enrobés dans la résine d'encapsulation 24.

Selon le mode de réalisation illustré à la figure 3, les électrodes 18 sont de forme circulaire. Dans une variante, la forme des électrodes 18 peut être différente, par exemple de forme carrée, rectangulaire, ovale, triangulaire, en spirale, en croix ou en étoile.

Préférentiellement, les électrodes 18, 18A, 18B ont une surface de contact comprise entre 0,5 mm² et 8 mm².

Les électrodes 18 sont agencées par couple d'électrodes 30.

Un couple d'électrodes 30 est formée par deux électrodes 18A, 18B agencées à proximité l'une de l'autre sans se toucher. Les électrodes 18A, 18B constituant un couple d'électrodes 30 sont espacées l'une de l'autre par une distance inter-électrodes d1. La distance d1 peut être variée selon le mode de réalisation. En particulier, la distance d1 est comprise entre 100 micromètres et 2 millimètres. La distance inter-électrodes d1 dans un couple d'électrodes 30 peut varier entre les différents couples d'électrodes 30 du capteur d'humidité 10.

Tel qu'il sera expliqué plus en détail en référence à la figure 4, un couple d'électrodes 30 est associé à chaque moyen de détection 20.

Dans l'exemple illustré à la figure 3, la pluralité des électrodes 18 est disposée sur la première face 14 en croix, de manière à former une croix à six branches. Dans une variante (non représenté), la pluralité des électrodes 18 est disposée selon une configuration de croix à huit branches. Le nombre de branches n'est pas limitatif.

La configuration en croix permet de faciliter la diffusion et la propagation du liquide (indiquées par les flèches E en pointillé sur la figure 3) provenant du centre dans le plan (XZ) de la première face 14, notamment d'une zone centrale C de la première face 14 vers des zones périphériques P1, P2, P3, P4 de la première face 14.

D'autres dispositions des électrodes 18 pour détecter la diffusion du liquide dans toutes les directions du plan sont possibles (en ronds, concentriques etc.).

Tel qu'illustré à la Figure 3, la première face 14 comprend en outre une antenne radioélectrique 32. Le fait que l'antenne radioélectrique 32 soit disposée sur la première face 14, c'est-à-dire sur la face 14 qui est aussi pourvue avec les électrodes 18, permet non seulement un gain de place dans le capteur d'humidité 10 mais aussi de faciliter le montage et les connexions électriques du substrat de support 12.

L'antenne radioélectrique 32 est électriquement reliée au circuit imprimé 22 de la deuxième face 16 par au moins un trou métallisé (non visible sur la Figure 3). En particulier, l'antenne peut être connectée électriquement à un circuit intégré du module de communication COM.

Dans l'exemple de la Figure 3, l'antenne radioélectrique 32 a une forme de spirale dont les bras passent entre les électrodes 18.

Dans une variante, illustrée à la Figure 4, le capteur d'humidité 10 peut être pourvu d'une l'antenne radioélectrique 34 qui est disposée autour de la pluralité des couples d'électrodes 30. L'antenne radioélectrique 34 est électriquement reliée au circuit imprimé 22 de la deuxième face 16 par au moins un trou métallisé (non visible sur la Figure 4). La disposition selon l'exemple de la Figure 4 permet de s'affranchir plus facilement du facteur de forme à prendre en considération lors de la fabrication de capteur d'humidité 10 de différentes dimensions dans le plan (XZ).

Les antennes 32, 34 permettent une connexion sans fil avec un dispositif tiers (non représenté) du capteur d'humidité 10, notamment au moyen d'un circuit intégré du module COM qui sera connecté aux antennes 32, 34.

Il est noté que seule l'antenne diffère entre les représentations du capteur d'humidité 10 représenté aux Figures 3 et 4.

Le capteur d'humidité 10 est sensible à l'étendue de la première face 14 mouillée par un liquide. Au fur et à mesure que le liquide s'écoule, il se répand sur la première face 14, par exemple depuis la zone centrale C. Ainsi, du liquide 36 peut être présent entre deux électrodes 18A, 18B d'un couple d'électrodes 30. La présence de liquide 36 entre deux électrodes 18A, 18B d'un couple d'électrodes 30 connecte électriquement les électrodes 18A, 18B entre elles. Le passage d'un courant électrique entre les électrodes 18A, 18B génère un court-circuit entre lesdites électrodes 18A, 18B du couple d'électrodes 30. La mise en court-circuit des électrodes 18A, 18B est utilisée par les moyens de détection 20 pour déterminer la présence et la répartition de liquide sur la première face 14, tel que décrit davantage ci-après en référence aux figures suivantes.

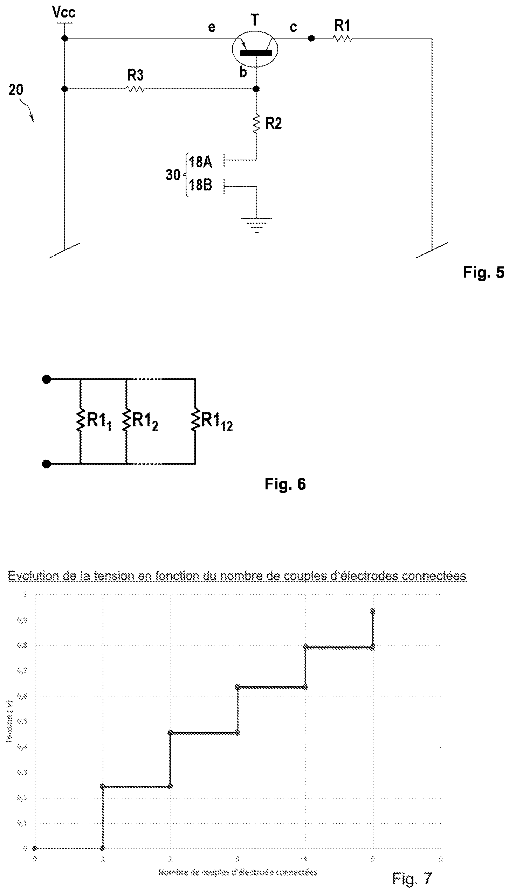

La Figure 5 représente schématiquement un moyen de détection 20 selon un mode de réalisation de la présente invention.

Les éléments avec les mêmes références numériques déjà utilisées pour la description de la Figures 1 à 4 ne seront pas décrits à nouveau en détail, et référence est faite à leurs descriptions ci-dessus.

Chaque moyen de détection 20 selon la présente invention comprend un transistor T associé à un couple d'électrodes 30 formé par deux électrodes 18A, 18B de la pluralité d'électrodes 18.

En particulier, le transistor T est un transistor bipolaire de type positif-négatif-positif (PNP).

Dans une variante, le transistor T est un transistor à effet de champ à grille isolée (« metal-oxide-semiconductor field-effect transistor » ou MOSFET en anglais), et l'on pourra adapter le circuit décrit ci-après en conséquence. En particulier, la base, le collecteur, et l'émetteur du transistor bipolaire correspondent respectivement à la grille, à la source, et au drain du transistor MOSFET.

Le transistor T est configuré pour commuter d'un état premier, dans lequel le transistor T est passant vers un second état, dans lequel le transistor T est non-passant (ou bloqué). En fait, le transistor T opère dans la zone de saturation.

L'émetteur e du transistor T est relié à la tension d'alimentation Vcc.

Le collecteur c du transistor T est connecté en série à une première résistance R1, par exemple une résistance R1 de 1,2 M Ohm. Les premières résistances R1 de chaque moyen de détection 20 sont connectées en parallèle entre elles dans le circuit électrique 22.

Dans une variante, les premières résistances R1 du capteur d'humidité 10 ont des valeurs différentes. Par exemple, la valeur des premières résistances R1 disposées au niveau de la zone centrale C (voir Figures 3 et 4) de la première face 14 est différente de la valeur des premières résistances R1 disposées vers les zones périphériques P1, P2, P3, P4 (voir Figures 3 et 4) de la première face 14.

La base b du transistor T est reliée à une deuxième résistance R2, par exemple une résistance R2 de 20K Ohm. La résistance R2 sert à faire changer l'état du transistor T lorsque le liquide contacte les électrodes 18A, 18B.

La deuxième résistance R2 est connectée en série à une électrode 18A du couple d'électrodes 30.

L'autre électrode 18B du couple d'électrodes 30 est mise à la masse, c'est-à-dire à un potentiel de référence, généralement à 0 Volt.

Une troisième électrode R3, par exemple une résistance R3 de 200K Ohm, est reliée à la tension d'alimentation Vcc et la base du transistor T, tel que représenté sur la figure 5.

Il est noté que le moyen de détection 20 selon la présente invention n'est pas strictement limité au schéma du circuit électrique du mode de réalisation illustré par la figure 5.

Dans le mode de réalisation illustré à la figure 5, le transistor T est à l'état passant lorsque les électrodes 18A, 18B sont sèches, c'est-à-dire lorsque qu'un courant électrique ne peut pas circuler à travers le couple d'électrode 30, en l'absence de conducteur entre les électrodes 18A, 18B.

Le transistor T commute à l'état non-passant lorsque du liquide connecte les électrodes 18A, 18B, mettant ainsi la base b du transistor T à la masse. En effet, la présence de liquide entre les électrodes 18A, 18B de chaque couple d'électrodes 30 modifie la tension jusqu'à ce que la tension atteigne un seuil de tension auquel le transistor T est configuré pour commuter automatiquement d'un premier état vers un deuxième état, en particulier d'un état passant vers un étant non-passant. Le transistor T fonctionne ainsi en commutation, c'est-à-dire un type de fonctionnement « tout ou rien » (ou d'interrupteur). La tension aux bornes de la première résistance R1 en série avec le collecteur du transistor T varie ainsi d'un pallier à un autre, selon l'état du transistor T, en opposition à une évolution progressive de la tension.

La tension aux bornes de la première résistance R1 de chaque moyen de détection 20 est donc représentative de l'état du transistor T correspondant. L'état (passant ou non-passant/bloqué) du transistor T est quant à lui représentatif de la présence de liquide (ou non) entre le couple d'électrodes 30 de chaque moyen de détection 20. Les premières résistances R1 sont montées en parallèle dans le circuit électrique 22.

La Figure 6 représente schématiquement l'association en parallèle des premières résistances R1 du capteur d'humidité 10 qui comprend dans l'exemple illustré aux Figures 3 et 4, douze résistances R1 (R1₁ à R1₁₂) en parallèle et vingt-quatre électrodes 18 formant douze couples d'électrodes 30. Le nombre d'électrodes, et donc de résistances R1, n'est pas limitatif.

Afin de déterminer la présence et la répartition de liquide sur la face 14, le capteur d'humidité 10 est configuré pour mesurer la résistance équivalente Réq des premières résistances R1 en parallèle. La résistance équivalente Réq des premières résistances R1 en parallèle peut être mesurée à intervalle de temps prédéfini. De la résistance équivalente Réq peut être déduit le nombre de première résistance R1 associée à un transistor T qui a été commuté par la présence de liquide entre les électrodes 18A, 18B d'un couple d'électrodes 30. Il est ainsi possible d'en déduire le nombre de couples d'électrodes 30 connectées par la présence de liquide 34 entre les électrodes 18A, 18B du couple d'électrodes 30. Le nombre de couples d'électrodes 30 connectées est relatif à la surface mouillée par le liquide 36.

La Figure 7 montre une représentation graphique de l'évolution de la tension en fonction du nombre de couples d'électrodes 30 connectées par la présence de liquide 36 entre les électrodes 18A, 18B du couple d'électrodes 30. Il peut être constaté que la tension augmente par palier à chaque couple d'électrodes connectées (indiqué en abscisse). L'évolution de la tension par palier est notamment dépendante de la valeur des résistances R2 et R3.

Le capteur d'humidité 10 est ainsi sensible au nombre de transistor T commutés dans un état passant (respectivement dans un état bloqué) par rapport à ceux commutés dans un état bloqué (respectivement dans un état passant). Le capteur d'humidité 10 selon la présente invention fonctionne indépendamment de la valeur de la conductivité électrique du liquide entre les électrodes 18A, 18B de chaque couple d'électrodes 30.

Ainsi, une méthode de mesure d'humidité au moyen capteur d'humidité 10 selon la présente invention consiste à mesurer la résistance équivalente Réq des premières résistances R1 qui est dépendante du nombre de transistors T commutés, et de déduire de la valeur de la résistance équivalente mesurée la présence de liquide sur la première face 14 pourvue des électrodes 18.

Il est noté que les transistors T sont compatibles avec une puce électronique, notamment pour mesurer la résistance équivalente Réq et traiter les données mesurées. La puce électronique peut être configurée pour transmettre des données à un dispositif tiers par une communication radiofréquence ou une communication en champ proche (« Near Field Communication » ou NFC en anglais), notamment au moyen de l'antenne 32 ou 34. Ainsi, le capteur d'humidité 10 est adapté pour pouvoir traiter les données recueillies par les moyens de détections et les communiquer par radiofréquence. Ces données comprennent au moins une information relative à l'humidité détectée par le capteur d'humidité 10, par exemple une valeur d'humidité, une valeur relative à la surface de la première face 14 recouverte par du liquide (par exemple un nombre d'électrode), ou encore un pourcentage relatif à l'humidité.

Dans le cas où le capteur d'humidité 10 est intégré dans un pansement 100, tel qu'illustré à la Figure 2, le capteur d'humidité 10 permet avantageusement de détecter la présence et l'étendue de l'exsudat dans la couche absorbante 102 du pansement 100 selon le principe décrit en référence aux Figures 5 à 7.

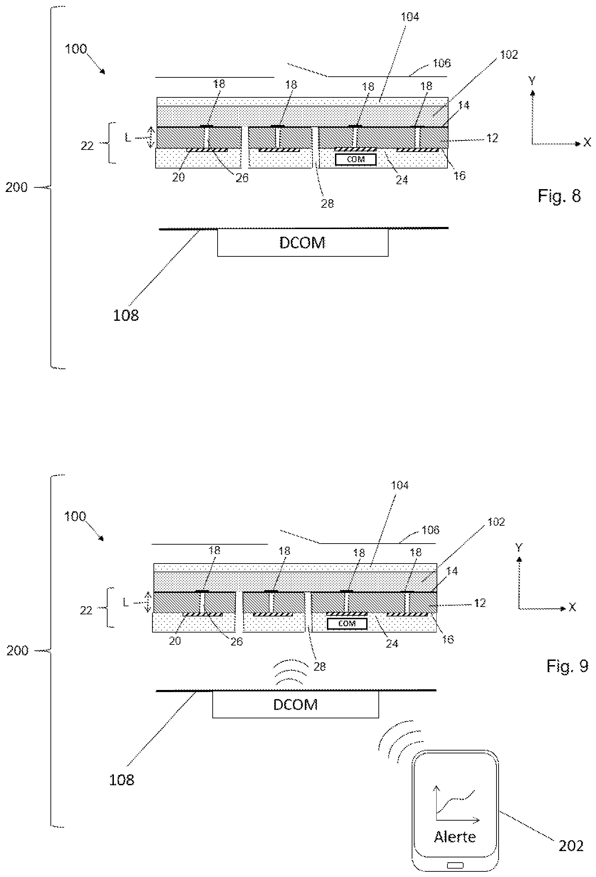

La Figure 8 représente un système 200 comprenant un pansement 100, tel qu'illustré à la Figure 2, et un dispositif de communication DCOM.

Le pansement 100 comprend en outre une couche de protection 108, fournie du côté opposé à la face d'application 104. La couche de protection 108 peut être également désignée par l'homme du métier comme une couche de support 108. C'est cette couche 108 qui, dans un pansement, est imperméable à l'eau mais perméable à l'air, d'une manière connue de l'homme du métier.

Dans le système 200, un dispositif de communication DCOM est fixé de manière amovible sur la couche de protection 108 du pansement 100, par exemple au moyen d'un adhésif, d'un système textile à crochets et boucles, ou d'une poche. Ainsi, le dispositif de communication DCOM peut être détaché pour être réutilisé, tandis que le pansement 100 sera jeté après avoir été utilisé. Le dispositif de communication DCOM, étant amovible, agit comme un « patch » qui sera fixé uniquement lorsque le système 200 est sur un utilisateur, puis conservé pour un autre pansement 100 lorsque celui-ci est changé. Cela limite la quantité de déchets électroniques.

Le dispositif de communication DCOM peut entrer en communication avec le module de communication COM par communication en champ proche, par exemple pour récupérer les informations relatives à l'humidité et les mémoriser dans une mémoire (non représentée) du dispositif de communication DCOM.

Le dispositif de communication DCOM comprend une batterie d'alimentation électrique (non représentée), et alimente le pansement 100 du système 200 en énergie électrique par induction magnétique.

Comme illustré sur la Figure 9, le dispositif de communication DCOM peut par ailleurs être capable de communiquer avec un terminal utilisateur 202 (par exemple par une communication selon la norme Bluetooth (*Low Energy*)), de sorte qu'une donnée relative à l'humidité détectée par le capteur d'humidité du pansement 100 du système 200 peut être reçue et affichée sur le terminal utilisateur 202.

A titre indicatif, le dispositif de communication DCOM peut transmettre un signal d'alerte qui sera affiché sur le terminal utilisateur 202, par exemple pour indiquer qu'une vitesse de variation d'humidité dépasse un seuil donné.

### Liste des références

10 : capteur d'humidité
12 : substrat de support
14 : première face
16 : deuxième face
18 : électrodes
20 : moyens de détection
22 : circuit imprimé électrique
24 : résine d'encapsulation
26 : trou métallisé
28 : orifice traversant
30 : couple d'électrodes
32 : antenne
34 : antenne
36 : liquide
100 : pansement
102 : couche absorbante
104 : face d'application
106 : film de protection détachable
108 : couche protectrice
200 : système
202 : terminal utilisateur
COM : module de communication
DCOM : dispositif de communication
b : base
c : collecteur
C : zone centrale
d1 : distance inter-électrodes
L : épaisseur
e : émetteur
E : direction écoulement
P1, P2, P3, P4 : zones périphériques
R1 : première résistance
R2, R3 : résistance
Réq : résistance équivalente
T : transistor
Vcc : tension d'alimentation
X, Y, Z : repère de coordonnées Cartésien

## Revendications

1. Capteur d'humidité (10) comprenant un substrat de support (12) ayant une première face (14) et une deuxième face (16), géométriquement opposée à la première face (14), et dans lequel :
la première face (14) est pourvue d'une pluralité de couples d'électrodes (30)
configurés pour être en contact avec un liquide,
le capteur d'humidité (10) étant **caractérisé en ce que**:
la deuxième face (16) comprend une pluralité de premiers moyens de détection (20) connectés entre eux en parallèle, et
chaque premier moyen de détection (20) comprend un transistor (T) associé à un couple d'électrodes (30), et
ledit transistor (T) de chaque premier moyen de détection (20) est configuré pour commuter d'un premier état vers un second état lors du passage d'un courant électrique entre des électrodes (18A, 18B) du couple d'électrodes (30) associé au transistor (T),
une électrode (18A) du couple d'électrodes (30) étant électriquement connectée en série à une électrode d'entrée (b) du transistor (T) et
l'autre électrode (18B) du couple d'électrodes (30) étant électriquement connectée à un potentiel fixe, en particulier à un potentiel à la masse, et
une électrode de sortie (c) du transistor (T) étant électriquement connecté en série avec une première résistance (R1),
de sorte que le capteur d'humidité (10) est sensible à la résistance équivalente (Réq) desdites premières résistances (R1) de la pluralité des premiers moyens de détection (20).

2. Capteur d'humidité selon la revendication 1, dont le transistor (T) est un transistor bipolaire (T), en particulier un transistor bipolaire de type positif-négatif-positif (PNP), configuré pour commuter d'un état dit passant, correspondant au premier état, vers un état dit bloqué, correspondant au second état, et dans lequel l'électrode d'entrée (b) du transistor (T) est une base (b) du transistor bipolaire (T) et l'électrode de sortie (c) du transistor (T) est un collecteur du transistor bipolaire (T).

3. Capteur d'humidité selon la revendication 1, dont le transistor (T) est un transistor à effet de champ à grille isolée (MOSFET) dans lequel l'électrode d'entrée (b) du transistor (T) est une grille du transistor MOSFET et l'électrode de sortie (c) du transistor (T) est un drain du transistor MOSFET.

4. Capteur d'humidité selon l'une des revendications précédentes, comprenant des premières résistances (R1) de valeurs différentes.

5. Capteur d'humidité selon la revendication 4, dont la pluralité de couples d'électrodes (30) est repartie par zones prédéfinies de la première face (14), et
la valeur de chaque première résistance (R1) est relative à une zone prédéfinie de la première face (14).

6. Capteur d'humidité selon l'une des revendications précédentes, dont la pluralité de premiers moyens de détection (20) est enrobée dans une résine d'encapsulation (24).

7. Capteur d'humidité selon l'une des revendications précédentes, dont le substrat de support (12) comprend au moins un orifice traversant (28) permettant un écoulement d'un fluide entre la première face (14) et la deuxième face (16).

8. Capteur d'humidité selon l'une des revendications précédentes, dont les électrodes (18, 18A, 18B) de la pluralité de couples d'électrodes (30) sont disposées sur la première face (14) selon au moins deux directions contenues dans un plan de la première face (14), en particulier en croix, en particulier en croix à au moins six branches.

9. Capteur d'humidité selon l'une des revendications précédentes, dans lequel, pour chaque couple d'électrodes (30), chacune des électrodes (18, 18A, 18B) du couple d'électrodes (30) est électriquement connectée par un trou métallisé (26) formé entre la première face (14) et la deuxième face (16) à un premier moyen de détection (20) correspondant qui est disposé sur ladite deuxième face (16).

10. Capteur d'humidité selon l'une des revendications précédentes, dont le substrat de support (12) est une couche flexible apte à être courbée, en particulier de moins de 300 micromètres d'épaisseur, plus particulièrement de moins de 150 micromètres d'épaisseur.

11. Capteur d'humidité selon l'une des revendications précédentes, comprenant en outre une puce électronique au niveau de la deuxième face (16) configurée pour transmettre des données à un dispositif tiers.

12. Capteur d'humidité selon l'une des revendications précédentes, dont la première face (14) comprend en outre une antenne radioélectrique (32, 34).

13. Capteur d'humidité selon la revendication 12, dont l'antenne radioélectrique (34) est disposée autour de la pluralité des couples d'électrodes (30).

14. Capteur d'humidité selon l'une des revendications précédentes, dont le substrat de support (12) comprend en outre au moins un autre couple d'électrodes de détection et au moins un deuxième moyen de détection d'une autre grandeur que l'humidité, et ledit couple d'électrodes de détection étant électriquement connecté au deuxième moyen de détection de sorte que le capteur d'humidité (10) est en outre configuré pour mesurer une autre grandeur que l'humidité, en particulier la température, la pression, le pH ou la présence d'une espèce chimique.

15. Utilisation d'un capteur d'humidité (10) selon l'une des revendications précédentes, dans un pansement (100) de plaie dans lequel la pluralité de couples d'électrodes (30) du capteur d'humidité (10) est agencé dans le pansement (100) pour être en contact avec un liquide provenant d'une plaie.

16. Procédé de détection d'humidité au moyen d'un capteur d'humidité selon l'une des revendications 1 à 14, comprenant les étapes de :
a) mesurer la résistance équivalente (Réq) des premières résistances (R1) de la pluralité des premiers moyens de détection (20) du capteur d'humidité qui est dépendante du nombre de transistors (T) commutés dans un des premiers ou second états, puis
b) déduire de la valeur de la résistance équivalente mesurée la présence de liquide sur la première face (14) pourvue des électrodes (18).

## Patentansprüche

1. Feuchtigkeitssensor (10), der ein Trägersubstrat (12) mit einer ersten Seite (14) und einer zweiten Seite (16), die der ersten Seite (14) geometrisch gegenüberliegt, umfasst, und wobei:
die erste Seite (14) mit einer Vielzahl von Elektrodenpaaren (30) versehen ist, die konfiguriert sind, um mit einer Flüssigkeit in Kontakt zu kommen,
wobei der Feuchtigkeitssensor (10) **dadurch gekennzeichnet ist, dass**:
die zweite Seite (16) eine Vielzahl von ersten Detektionsmitteln (20) umfasst, die parallel zueinander geschaltet sind, und
jedes erste Detektionsmittel (20) einen Transistor (T) umfasst, der mit einem Elektrodenpaar (30) verbunden ist, und
der Transistor (T) jedes ersten Detektionsmittels (20) so konfiguriert ist, dass er von einem ersten Zustand in einen zweiten Zustand schaltet, wenn ein elektrischer Strom zwischen Elektroden (18A, 18B) des Elektrodenpaars (30), das dem Transistor (T) zugeordnet ist, hindurch fließt,
wobei eine Elektrode (18A) des Elektrodenpaars (30) elektrisch mit einer Eingangselektrode (b) des Transistors (T) in Reihe geschaltet ist und
wobei die andere Elektrode (18B) des Elektrodenpaars (30) elektrisch an ein festes Potential, insbesondere an ein Massepotential, angeschlossen ist, und eine Ausgangselektrode (c) des Transistors (T) elektrisch mit einem ersten Widerstand (R1) in Reihe geschaltet ist,
so dass der Feuchtigkeitssensor (10) empfindlich auf den äquivalenten Widerstand (Req) der ersten Widerstände (R1) der Vielzahl von ersten Detektionsmittel (20) reagiert.

2. Feuchtigkeitssensor nach Anspruch 1, dessen Transistor (T) ein Bipolartransistor (T) ist, insbesondere ein Bipolartransistor vom Typ positiv-negativ-positiv (PNP), der so konfiguriert ist, dass er von einem so genannten leitenden Zustand, der dem ersten Zustand entspricht, in einen so genannten gesperrten Zustand, der dem zweiten Zustand entspricht, umschaltet, und wobei die Eingangselektrode (b) des Transistors (T) eine Basis (b) des Bipolartransistors (T) ist und die Ausgangselektrode (c) des Transistors (T) ein Kollektor des Bipolartransistors (T) ist.

3. Feuchtigkeitssensor nach Anspruch 1, dessen Transistor (T) ein Feldeffekttransistor mit isoliertem Gate (MOSFET) ist, wobei die Eingangselektrode (b) des Transistors (T) ein Gate des MOSFET-Transistors ist und die Ausgangselektrode (c) des Transistors (T) ein Drain des MOSFET-Transistors ist.

4. Feuchtigkeitssensor nach einem der vorstehenden Ansprüche, der erste Widerstände (R1) mit unterschiedlichen Werten umfasst.

5. Feuchtigkeitssensor nach Anspruch 4, wobei die Vielzahl von Elektrodenpaaren (30) nach vordefinierten Bereichen auf der ersten Seite (14) verteilt ist und sich der Wert jedes ersten Widerstands (R1) auf einen vordefinierten Bereich der ersten Seite (14) bezieht.

6. Feuchtigkeitssensor nach einem der vorstehenden Ansprüche, wobei die Vielzahl von ersten Detektionsmitteln (20) in ein Verkapselungsharz (24) eingeschlossen ist.

7. Feuchtigkeitssensor nach einem der vorstehenden Ansprüche, wobei das Trägersubstrat (12) mindestens eine Durchgangsöffnung (28) umfasst, die einen Abfluss eines Fluids zwischen der ersten Seite (14) und der zweiten Seite (16) ermöglicht.

8. Feuchtigkeitssensor nach einem der vorstehenden Ansprüche, wobei die Elektroden (18, 18A, 18B) der Vielzahl von Elektrodenpaaren (30) auf der ersten Seite (14) in mindestens zwei Richtungen, die in einer Ebene der ersten Seite (14) enthalten sind, insbesondere kreuzförmig, insbesondere als Kreuz mit mindestens sechs Armen, angeordnet sind.

9. Feuchtigkeitssensor nach einem der vorstehenden Ansprüche, wobei für jedes Elektrodenpaar (30) jede der Elektroden (18, 18A, 18B) des Elektrodenpaars (30) durch ein zwischen der ersten Seite (14) und der zweiten Seite (16) ausgebildetes metallisiertes Loch (26) elektrisch an ein entsprechendes erstes Detektionsmittel (20) angeschlossen ist, das auf der zweiten Seite (16) angeordnet ist.

10. Feuchtigkeitssensor nach einem der vorstehenden Ansprüche, wobei das Trägersubstrat (12) eine flexible, verbiegbare Schicht ist, insbesondere mit einer Dicke von weniger als 300 Mikrometern, bevorzugt mit einer Dicke von weniger als 150 Mikrometern.

11. Feuchtigkeitssensor nach einem der vorstehenden Ansprüche, der außerdem einen Mikrochip auf der zweiten Seite (16) umfasst, der so konfiguriert ist, dass er Daten an ein Drittgerät überträgt.

12. Feuchtigkeitssensor nach einem der vorstehenden Ansprüche, wobei die erste Seite (14) zusätzlich eine Funkantenne (32, 34) umfasst.

13. Feuchtigkeitssensor nach Anspruch 12, wobei die Funkantenne (34) um die Vielzahl von Elektrodenpaaren (30) herum angeordnet ist.

14. Feuchtigkeitssensor nach einem der vorstehenden Ansprüche, wobei dessen Trägersubstrat (12) zusätzlich mindestens ein weiteres Detektionselektrodenpaar und mindestens ein zweites Detektionsmittel für eine andere Größe als Feuchtigkeit umfasst, und wobei das Detektionselektrodenpaar elektrisch an das zweite Detektionsmittel angeschlossen ist, so dass der Feuchtigkeitssensor (10) zusätzlich so konfiguriert ist, dass er eine andere Größe als Feuchtigkeit messen kann, insbesondere Temperatur, Druck, pH-Wert oder das Vorhandensein einer chemischen Substanz.

15. Verwendung eines Feuchtigkeitssensors (10) nach einem der vorstehenden Ansprüche in einem Wundverband (100), wobei die Vielzahl von Elektrodenpaaren (30) des Feuchtigkeitssensors (10) in dem Verband (100) angeordnet ist, um mit einer Flüssigkeit aus einer Wunde in Kontakt zu kommen.

16. Verfahren zum Detektieren von Feuchtigkeit mithilfe eines Feuchtigkeitssensors nach einem der Ansprüche 1 bis 14, das die folgenden Schritte umfasst:
a) Messen des äquivalenten Widerstands (Req) der ersten Widerstände (R1) der Vielzahl von ersten Detektionsmitteln (20) des Feuchtigkeitssensors, der von der Anzahl von Transistoren (T) abhängt, die in einen der ersten oder zweiten Zustände geschaltet sind, und anschließend
b) Ableiten aus dem Wert des gemessenen äquivalenten Widerstands das Vorhandensein von Flüssigkeit auf der ersten, mit den Elektroden (18) versehenen Seite (14).

## Claims

1. A moisture sensor (10) comprising a support substrate (12) having a first face (14) and a second face (16), geometrically opposite to the first face (14), and wherein:
the first face (14) is provided with a plurality of pairs of electrodes (30) configured to be in contact with a liquid,
the moisture sensor (10) being **characterized in that**:
the second face (16) comprises a plurality of first means of detection (20) connected to each other in parallel, and
each first means of detection (20) comprises a transistor (T) associated with a pair of electrodes (30), and
said transistor (T) of each first means of detection (20) is configured to switch from a first state to a second state when an electric current passes between electrodes (18A, 18B) of the pair of electrodes (30) associated to the transistor (T),
an electrode (18A) of the pair of electrodes (30) being electrically connected in series to an input electrode (b) of the transistor (T) and
the other electrode (18B) of the pair of electrodes (30) being electrically connected to a fixed potential, in particular, to an earth potential, and
an output electrode (c) of the transistor (T) being electrically connected in series with a first resistor (R1),
such that the moisture sensor (10) is sensitive to the equivalent resistance (Req) of said first resistors (R1) of the plurality of the first means of detection (20).

2. The moisture sensor according to claim 1, wherein the transistor (T) is a bipolar transistor (T), in particular, a positive-negative-positive (PNP) bipolar transistor, configured to switch from a so-called on-state, corresponding to the first state, to a so-called off-state, corresponding to the second state, and wherein the input electrode (b) of the transistor (T) is a base (b) of the bipolar transistor (T) and the output electrode (c) of the transistor (T) is a collector of the bipolar transistor (T).

3. The moisture sensor according to claim 1, wherein the transistor (T) is a Metal Oxide Semiconductor Field Effect Transistor (MOSFET) wherein the input electrode (b) of the transistor (T) is a gate of the MOSFET and the output electrode (c) of the transistor (T) is a drain of the MOSFET.

4. The moisture sensor according to one of the preceding claims, comprising first resistors (R1) of different values.

5. The moisture sensor according to claim 4, wherein the plurality of pairs of electrodes (30) is distributed by predefined zones of the first face (14), and the value of each first resistor (R1) is relative to a predefined zone of the first face (14).

6. The moisture sensor according to one of the preceding claims, wherein the plurality of the first means of detection (20) is encased in an encapsulation resin (24).

7. The moisture sensor according to one of the preceding claims, wherein the support substrate (12) comprises at least one through orifice (28) allowing a fluid to flow between the first face (14) and the second face (16).

8. The moisture sensor according to one of the preceding claims, wherein the electrodes (18, 18A, 18B) of the plurality of pairs of electrodes (30) are arranged on the first face (14) in at least two directions contained in a plane of the first face (14), in particular, cross-shaped, in particular, cross-shaped with at least six points.

9. The moisture sensor according to one of the preceding claims, wherein, for each pair of electrodes (30), each of the electrodes (18, 18A, 18B) of the pair of electrodes (30) is electrically connected by a metallised hole (26) formed between the first face (14) and the second face (16) to a corresponding first means of detection (20) that is arranged on said second face (16).

10. The moisture sensor according to one of the preceding claims, wherein the support substrate (12) is a flexible layer capable of being curved, in particular, less than 300 micrometres thick, more particularly, less than 150 micrometres thick.

11. The moisture sensor according to one of the preceding claims, further comprising a microchip at the second face (16) configured to transmit data to a third-party device.

12. The moisture sensor according to one of the preceding claims, wherein the first face (14) further comprises a radio antenna (32, 34).

13. The moisture sensor according to claim 12, wherein the radio antenna (34) is arranged around the plurality of pairs of electrodes (30).

14. The moisture sensor according to one of the preceding claims, wherein the support substrate (12) further comprises at least another pair of detecting electrodes and at least a second means of detection of a quantity other than moisture, and said pair of detecting electrodes being electrically connected to the second means of detection such that the moisture sensor (10) is further configured to measure a quantity other than moisture, in particular, temperature, pressure, pH, or the presence of a chemical species.

15. Use of a moisture sensor (10) according to one of the preceding claims, in a wound dressing (100), wherein the plurality of pairs of electrodes (30) of the moisture sensor (10) is arranged in the wound dressing (100) to be in contact with a liquid from a wound.

16. Method for detecting moisture by means of a moisture sensor according to one of claims 1 to 14, comprising the following steps of:
a) measuring the equivalent resistance (Req) of the first resistors (R1) of the plurality of the first means of detection (20) of the moisture sensor that is dependent on the number of transistors (T) switched in one of the first or second states, then,
b) from the equivalent resistance value measured, deducing the presence of liquid on the first face (14) provided with the electrodes (18).
